# EUROPÄISCHE PATENTSCHRIFT

(11) **EP 2 026 761 B1**
(45) Veröffentlichungstag und Bekanntmachung des Hinweises auf die Patenterteilung: **15.04.2020**
(21) Anmeldenummer: 07728941.1
(22) Anmeldetag: 09.05.2007
(51) Int. Cl.: A61K 9/00, A61K 31/47, A61P 11/06, A61P 11/08

(54) **TREIBGASFREIE AEROSOLFORMULIERUNG FÜR DIE INHALATION**
INHALANT PROPELLANT-FREE AEROSOL FORMULATION
FORMULATION D'AÉROSOL EXEMPTE DE GAZ PROPULSEUR, DESTINÉE À ÊTRE INHALÉE

(30) Priorität: 20.05.2006 DE 102006023770
(43) Veröffentlichungstag der Anmeldung: 25.02.2009
(73) Patentinhaber: Boehringer Ingelheim International GmbH, 55216 Ingelheim am Rhein (DE); Boehringer Ingelheim Pharma GmbH & Co. KG, 55216 Ingelheim am Rhein (DE)
(72) Erfinder: KREHER, Christoph, 55218 Ingelheim/Rhein (DE); SPALLEK, Michael, 55218 Ingelheim am Rhein (DE)
(74) Vertreter: Simon, Elke Anna Maria
(86) Internationale Anmeldenummer: PCT/EP2007/054489
(87) Internationale Veröffentlichungsnummer: WO 2007/134966

(56) Entgegenhaltungen:
- WO-A-03/035030
- WO-A-2004/022132
- WO-A-2004/024157
- US-A1- 2004 019 073

## Beschreibung

Die vorliegende Erfindung betrifft treibgasfreie Aerosolformulierungen für die Inhalation enthaltend einen oder mehrere inerte, nichtflüchtige Hilfsstoffe zur Einstellung definierter Tröpfchengrößen.

### Hintergrund der Erfindung

Die inhalative Applikation von Arzneimitteln gelangt nicht ausschließlich, aber insbesondere bei der Therapie von Atemwegserkrankungen wie beispielsweise Asthma oder COPD zur Anwendung. Geeignete Arzneimittelformulierungen sind neben Pulverformulierungen und treibgashaltigen Aerosolformulierungen vor allem auch treibgasfreie wässerige oder wässerig-alkoholische Lösungen von Wirkstoffen.

Solche treibgasfreien Lösungsformulierungen sind im Stand der Technik bekannt. Ethanolische Formulierungen werden beispielsweise durch die WO 97/01329 offenbart, wässerige Systeme werden beispielsweise durch die WO 98/27959 beschrieben.

Die WO03035030 offenbart flüssigen Zusammensetzungen, die als Aerosole verabreicht werden können, umfassend einen oder mehrere Wirkstoffe und einen wasserlöslichen Hilfsstoff mit niedrigem Molekulargewicht. Die WO2004022132 betrifft pharmazeutische Formulierungen enthaltend ein inertes Trägermaterial geeignet zur oralen Inhalation. Die WO2004024157 und US2004019073 offenbaren pharmazeutische Zusammensetzungen enthaltend Tiotropiumsalze und pharmazeutisch verträgliche Zusatzstoffe.

Bei der inhalativen Applikation von Arzneimitteln kommt unabhängig von der Wahl der Formulierung der Größe der zu inhalierenden Partikel besondere Bedeutung zu. Nur Partikel eines bestimmten Größenbereichs können an den beabsichtigten Wirkort, die tiefen Verästelungen der Lunge, gelangen um dort den gewünschten therapeutischen Effekt zu entfalten. Die zu inhalierenden Partikel oder Teilchen sollten einerseits eine bestimmte Obergrenze nicht überschreiten. Zwecks Optimierung der Lungendeposition ist es zudem wünschenswert, dass die Teilchengröße eines Aerosols aber auch nicht unter eine bestimmte Untergrenze fällt. Bevorzugt sollte die Partikelgröße des Aerosols größer 0,5µm, für alveolare Deposition bevorzugt größer 1µm und für bronchiale Deposition besonders bevorzugt größer 2 µm sein.

Insbesondere leichtflüchtige Aerosole haben die Eigenschaft, dass deren Tröpfchen bei der Inhalation verdunsten und ihr Durchmesser dabei stark abnimmt.

Es ist Aufgabe der vorliegenden Erfindungen, Aerosollösungsformulierungen bereit zustellen, bei denen die minimale Teilchengröße der ausgebrachten Aerosolbestandteile nach unten begrenzt ist.

### Detaillierte Beschreibung der Erfindung

Überraschenderweise wurde gefunden, dass die vorstehend genannte Aufgabe gelöst werden kann, wenn den Aerosollösungsformulierungen inerte, nichtflüchtige Hilfsstoffe zugesetzt werden.

Dementsprechend betrifft die vorliegende Erfindung treibgasfreie Lösungsformulierungen für die Inhalation, die neben einem oder mehreren Wirkstoffen in einem Lösungsmittel ausgewählt aus der Gruppe Ethanol und Wasser-Ethanol-Gemisch, wenigstens einen inerten, nichtflüchtigen Hilfsstoff in einer solchen Menge enthalten, dass die Gesamtkonzentration an nichtflüchtigen Bestandteilen in der Formulierung > 3 Gew.-% beträgt.

Insbesondere betrifft die vorliegende Erfindung die Verwendung einer Lösungsformulierung zur Herstellung eines Arzneimittels zur Behandlung von Atemwegserkrankungen, wobei die Lösungsformulierungen neben einem oder mehreren Wirkstoffen aus der Gruppe bestehend aus Anticholinergika, Betamimetika, Corticosteroide, PDE4-Inhibitoren, LTD4-Antagonisten, Dopamin-Agonisten und H1-Antihistaminika in einem Lösungsmittel ausgewählt aus der Gruppe Ethanol oder Mischungen aus Ethanol und Wasser, wenigstens einem inerten, nichtflüchtigen Hilfsstoff, ausgewählt aus der Gruppe bestehend aus Natriumchlorid, Magnesiumsulfat, Natriumhydrogenkarbonat, Kaliumhydrogenkarbonat und Magnesiumchlorid in einer solchen Menge enthalten, dass die Gesamtkonzentration an nichtflüchtigen Bestandteilen in der Formulierung ≥ 3 Gew.-% beträgt.

Zur Gesamtkonzentration an nichtflüchtigen Bestandteilen tragen im Sinne der vorliegenden Erfindung alle suspendierten oder gelösten Bestandteile mit bei, die nach Verdampfen des Lösungsmittels verbleiben würden. Dies sind neben den Wirkstoffbestandteilen und gegebenenfalls enthaltenen Konservierungsstoffen und Komplexbildnern vor allem inerte, nichtflüchtige Hilfsstoffe.

Im Rahmen der erfindungemäßen Formulierungen sind inerte, nichtflüchtige Hilfsstoffe Verbindungen, die im Lösungsmittel der erfindungsgemäßen Formulierungen gelöst, teilweise gelöst oder suspendiert vorliegen können. Als inerte, nichtflüchtige Hilfsstoffe, seien beispielsweise genannt Monosaccharide, Disaccharide, Oligo- und Polysaccharide, Polyalkohole oder auch Salze.

Monosaccharide sind bevorzugt ausgewählt aus der Gruppe bestehend aus Glucose und Arabinose, wobei Glucose besonders bevorzugt ist. Disaccharide sind bevorzugt ausgewählt aus der Gruppe bestehend aus Lactose, Saccharose, Maltose und Trehalose, wobei von den Disacchariden Lactose besonders bevorzugt ist. Oligo- und Polysaccharide sind bevorzugt ausgewählt aus der Gruppe bestehend aus Dextranen. Polyalkohole sind bevorzugt ausgewählt aus der Gruppe bestehend aus Sorbitol, Mannitol, Xylit und Glycerin wobei von den Polyalkoholen Glycerin besonders bevorzugt ist. Salze sind bevorzugt ausgewählt aus der Gruppe bestehend aus Kaliumchlorid, Magnesiumchlorid, Magnesuimsulfat, Natriumchlorid, Natriumcitrat, Natriumphosphat, Natriumhydrogenphosphat, Natriumhydrogenkarbonat, Kaliumcitrat, Kaliumphosphat, Kaliumhydrogenphosphat, Kaliumhydrogencarbonat Calciumcarbonat und Calciumchlorid, wobei von den Salzen besonders Natriumchlorid, Magnesuimsulfat, Natriumhydrogenkarbonat, Kaliumhydrogenkarbonat und Magnesiumchlorid, insbesondere Natriumchlorid, Magnesuimsulfat und Natriumhydrogenkarbonat, bevorzugt sind.

Die erfindungsgemäßen Formulierungen können ferner etherische Öle als mukoaktive Substanzen auch zur Geschmacksmaskierung enthalten. Diesbezüglich von besonderem Interesse sind Eukalyptusöl, Edeltannenöl, Kiefernnadelöl oder auch Pfefferminzöl.

Besonders bevorzugt gelangen als inerte, nichtflüchtige Hilfsstoffe Verbindungen zum Einsatz, die ausgewählt sind aus der Gruppe der vorstehend genannten Salze, wobei den nachstehenden Hilfsstoffen besondere Bedeutung zukommt: Natriumchlorid. Natriumchlorid, Magnesuimsulfat. Natriumhydrogenkarbonat, Kaliumhydrogenkarbonat und Magnesiumchlorid.

Die Gesamtkonzentration an nichtflüchtigen Bestandteilen der erfindungsgemäßen Lösungsformulierungen ist ≥ 3 Gew-%, besonders bevorzugt ≥ 5 Gew-%, ferner bevorzugt ≥ 10 Gew-%. Die Bezeichnung " ≥ " steht dabei für "gleich oder mehr" wie beispielsweise "3 Gew-% oder mehr".
Die maximale Konzentration an nichtflüchtigen Bestandteilen sollte dabei einen Bereich von 20 Gew-% nicht überschreiten.

Besonders bevorzugt sind Arzneimittelzubereitungen, die nach Vernebelung durch einen geeigneten Inhalator Partikelgrößen von ≥ 0,5µm, für alveolare Deposition bevorzugt von ≥ 1 µm und für bronchiale Deposition besonders bevorzugt von ≥ 2 µm aufweisen.

Die erfindungsgemäßen Arzneimittelformulierungen enthalten als Lösungsmittel reines Ethanol oder Mischungen aus Ethanol und Wasser. Werden Ethanol-Wasser-Mischungen verwendet, so liegt der prozentuale Massenanteil von Ethanol in diesen Mischungen bevorzugt im Bereich zwischen 5 und 99 % Ethanol, besonders bevorzugt im Bereich von 10 bis 96 % Ethanol. Ganz besonders bevorzugte Arzneimittelformulierungen im Sinne der vorliegenden Erfindung enthalten als Lösungsmittel reines Ethanol oder Ethanol-Wasser-Mischungen enthaltend zwischen 50 und 92 %, besonders bevorzugt zwischen 69 und 91% Ethanol.

Gegebenenfalls können neben Ethanol und Wasser weitere Co-Solventien eingesetzt werden. Diese sind bevorzugt ausgewählt aus der Gruppe der Alkohole oder Ether, wie beispielsweise Isopropanol oder Tetrahydrofuran. Erfindungsgemäß bevorzugt gelangt ein weiteres Lösungsmittel allerdings nicht zum Einsatz.

Üblicherweise enthalten die erfindungsgemäßen Formulierungen pharmakologisch verträgliche Säuren zur Einstellung des pH-Wertes. Der pH-Wert der erfindungsgemäßen Formulierung liegt erfindungsgemäß bevorzugt in einem Bereich von 2,0 und 6,5, bevorzugt zwischen 2,2 und 5,0, besonders bevorzugt zwischen etwa 2,5 und 4,5.

Als pharmakologisch verträgliche Säuren können anorganische oder organische Säuren zur Anwendung gelangen. Beispiele für bevorzugte anorganische Säuren sind ausgewählt aus der Gruppe bestehend aus Salzsäure, Bromwasserstoffsäure, Salpetersäure, Schwefelsäure und Phosphorsäure. Beispiele für besonders geeignete organische Säuren sind ausgewählt aus der Gruppe bestehend aus Ascorbinsäure, Zitronensäure, Äpfelsäure, Weinsäure, Maleinsäure, Bernsteinsäure, Fumarsäure, Essigsäure, Ameisensäure und Propionsäure. Bevorzugte anorganische Säuren sind Salzsäure und Schwefelsäure, wobei der Salzsäure erfindungsgemäß besondere Bedeutung zukommt. Unter den organischen Säuren sind Ascorbinsäure, Fumarsäure und Zitronensäure bevorzugt, wobei Zitronensäure erfindungsgemäß besonders bevorzugt ist. Gegebenenfalls können auch Gemische der genannten Säuren eingesetzt werden, insbesondere in Fällen von Säuren, die neben ihren Säuerungseigenschaften auch andere Eigenschaften, z.B. als Geschmackstoffe oder Antioxidantien besitzen, wie beispielsweise Zitronensäure oder Ascorbinsäure. Gegebenenfalls können auch pharmakologisch verträgliche Basen zum genauen Austitrieren des pH-Wertes eingesetzt werden. Als Basen eignen sich beispielsweise Alkalihydroxide und Alkalicarbonate. Bevorzugtes Alkaliion ist Natrium. Werden solche Basen verwendet, ist darauf zu achten, dass auch die daraus resultierenden Salze, die dann in der fertigen Arzneimittelformulierung enthalten sind, mit der oben genannten Säure pharmakologisch akzeptabel sind.

Die erfindungsgemäßen Formulierungen können als weitere Bestandteile insbesondere Komplexbildner oder Konservierungsstoffe enthalten. Unter Komplexbildner werden im Rahmen der vorliegenden Erfindung Moleküle verstanden, die in der Lage sind Komplexbindungen einzugehen. Bevorzugt sollen durch diese Verbindungen Kationen, besonders bevorzugt metallische Kationen komplexiert werden. Die erfindungsgemäßen Formulierungen enthalten als Komplexbildner bevorzugt Editinsäure (EDTA) oder ein bekanntes Salze davon, z.B. Natrium-EDTA, bzw. Dinatrium-EDTA. Bevorzugt wird Dinatriumedetat, gegebenenfalls in Form seiner Hydrate, besonders bevorzugt in Form seines Dihydrats eingesetzt. Werden im Rahmen der erfindungsgemäßen Formulierungen Komplexbildner verwendet, so liegt deren Gehalt bevorzugt in einem Bereich von 1 bis 50 mg pro 100 ml, besonders bevorzugt in einem Bereich von 2 bis 15 mg pro 100 ml der erfindungsgemäßen Formulierung. Vorzugsweise enthalten die erfindungsgemäßen Formulierungen einen Komplexbildner in einer Menge von etwa 4 bis 12 mg pro 100 ml, besonders bevorzugt von etwa 10 mg pro 100 ml der erfindungsgemäßen Formulierung. Analoges wie bereits für Dinatriumedetat ausgeführt, gilt auch für mögliche, mit EDTA oder seinen Salzen vergleichbare Zusatzstoffe, die komplexbildende Eigenschaften aufweisen und anstelle dessen verwendet werden können, wie beispielsweise Nitrilotriessigsäure und deren Salze.

Konservierungsstoffe können eingesetzt werden, um die Formulierung vor Kontamination mit pathogenen Keimen zu schützen. Als Konservierungsstoffe eignen sich die dem Stand der Technik bekannten, insbesondere Benzalkoniumchlorid oder Benzoesäure bzw. Benzoate wie Natriumbenzoat in den aus dem Stand der Technik bekannten Konzentrationen. Bevorzugt wird der erfindungsgemäßen Formulierung Benzalkoniumchlorid beigemischt. Die Menge des Benzalkoniumchlorids beträgt dabei zwischen 1 mg und 50 mg pro 100 ml Formulierung, bevorzugt etwa 2 bis 15 mg pro 100 ml, besonders bevorzugt etwa 3 bis 12 mg pro 100 ml, besonders bevorzugt etwa 4 bis 10 mg pro 100 ml der erfindungsgemäßen Formulierung. Benzalkoniumchlorid kann erfindungsgemäß auch im Gemisch mit anderen Konservierungsstoffen zum Einsatz gelangen.

Die Wirkstoffe, die im Rahmen der erfindungsgemäßen Formulierungen zum Einsatz gelangen können sind ausgewählt aus der Gruppe bestehend aus der Gruppe der Anticholinergika, Betamimetika, Corticosteroide, PDE4-Inhibitoren, LTD4-Antagonisten, Dopamin-Agonisten und H1-Antihistaminika, wobei jeweils ein oder mehrere dieser Wirkstoffe enthalten sein können.

Anticholinergika, die in den erfindungsgemäßen Arzneimittelkombinationen als Wirkstoff eingesetzt werden können, sind bevorzugt ausgewählt aus der Gruppe bestehend aus Tiotropiumsalzen, Oxitropiumsalzen, Flutropiumsalzen, Ipratropiumsalzen, Glycopyrroniumsalzen und Trospiumsalzen. In den vorstehend genannten Salzen bis stellen die Kationen Tiotropium, Oxitropium, Flutropium, Ipratropium, Glycopyrronium und Trospium die pharmakologisch aktiven Bestandteile dar. Jede Bezugnahme auf die vorstehend genannten Salze schließt naturgemäß eine Bezugnahme auf die entsprechenden Kationen Tiotropium, Oxitropium, Flutropium, Ipratropium, Glycopyrronium, Trospium mit ein. Unter den Salzen bis werden erfindungsgemäß diejenigen Verbindungen verstanden, die neben den Kationen Tiotropium, Oxitropium, Flutropium, Ipratropium, Glycopyrronium und Trospium als Gegenion (Anion) Chlorid, Bromid, Iodid, Sulfat, Phosphat, Methansulfonat, Nitrat, Maleat, Acetat, Citrat, Fumarat, Tartrat, Oxalat, Succinat, Benzoat oder p-Toluolsulfonat enthalten, wobei Chlorid, Bromid, Iodid, Sulfat, Methansulfonat oder p-Toluolsulfonat als Gegenionen bevorzugt sind. Von allen Salzen sind die Chloride, Bromide, Iodide und Methansulfonate besonders bevorzugt.
Von besonderer Bedeutung sind Arzneimittelkombinationen, die Tiotropiumsalze, Oxitropiumsalze oder Ipratropiumsalze enthalten, wobei die jeweiligen Bromide erfindungsgemäß besonders bedeutsam sind. Von besonderer Bedeutung ist das Tiotropiumbromid. Die vorstehend genannten Salze können in den erfindungsgemäßen Arzneimittelkombinationen gegebenenfalls in Form ihrer Solvate oder Hydrate, bevorzugt in Form ihrer Hydrate vorliegen. Im Falle des Tiotropiumbromids enthalten die erfindungsgemäßen Arzneimittelkombinationen dieses bevorzugt in Form des kristallinen Tiotropiumbromid Monohydrats, welches aus der WO 02/30928 bekannt ist.

Gegebenenfalls weisen die vorstehend genannten Anticholinergika chirale Kohlenstoffzentren auf. In diesem Fall können die erfindungsgemäßen Arzneimittelkombinationen die Anticholinergika in Form ihrer Enantiomere, Mischungen der Enantiomere oder Racemate enthalten, wobei vorzugsweise enantiomerenreine Anticholinergika zum Einsatz gelangen.

Werden Tiotropiumsalze eingesetzt, liegt die Konzentration an Tiotropiumkation in den erfindungsgemäßen Arzneimittelformulierungen bevorzugt zwischen 0,01 g pro 100 g Formulierung und 0,06 g pro 100 g Formulierung. Bevorzugt ist eine Menge von 0,015 g /100 g bis 0,055 g / 100 g, stärker bevorzugt ist eine Menge von 0,02 g / 100 g bis 0,05 g / 100 g. Am stärksten bevorzugt ist eine Menge von 0,023 ± 0,001g pro 100 g Formulierung bis zu 0,045 ± 0,001g pro 100 g Formulierung.

Werden Ipratropiumsalze eingesetzt, liegt die Konzentration an Ipratropiumkation in den erfindungsgemäßen Arzneimittelformulierungen bevorzugt zwischen 0,20 g pro 100 g Formulierung und 1,58 g pro 100 g Formulierung. Bevorzugt ist eine Menge von 0,30 g /100 g bis 1,45 g / 100 g, stärker bevorzugt ist eine Menge von 0,40 g / 100 g bis 1,32 g / 100 g. Am stärksten bevorzugt ist eine Menge von 0,46 ± 0,02g pro 100 g Formulierung bis zu 0,92 ± 0,02 g pro 100 g Formulierung.

Werden Oxitropiumsalze eingesetzt, liegt die Konzentration an Oxitropiumkation in den erfindungsgemäßen Arzneimittelformulierungen bevorzugt zwischen 0,20 g pro 100 g Formulierung und 1,58 g pro 100 g Formulierung. Bevorzugt ist eine Menge von 0,30 g /100 g bis 1,45 g / 100 g, stärker bevorzugt ist eine Menge von 0,40 g / 100 g bis 1,32 g / 100 g. Am stärksten bevorzugt ist eine Menge von 0,46 ± 0,02g pro 100 g Formulierung bis zu 0,92 ± 0,02 g pro 100 g Formulierung.

In einer ebenfalls bevorzugten Ausführungsform der vorliegenden Erfindung sind die in den erfindungsgemäßen Zubereitungen enthaltenen Anticholinergika ausgewählt aus den Salzen der Formel 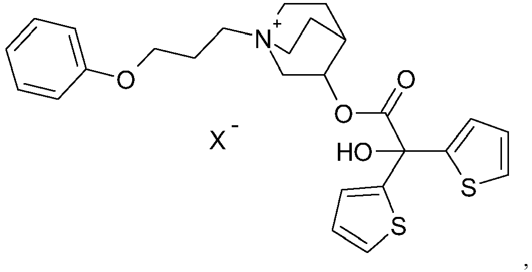 worin
X⁻ ein einfach negativ geladenes Anion, bevorzugt ein Anion ausgewählt aus der Gruppe bestehend aus Fluorid, Chlorid, Bromid, Iodid, Sulfat, Phosphat, Methansulfonat, Nitrat, Maleat, Acetat, Citrat, Fumarat, Tartrat, Oxalat, Succinat, Benzoat und p-Toluolsulfonat, bevorzugt Bromid bedeutet gegebenenfalls in Form ihrer Racemate, Enantiomere oder Hydrate.

Von besonderer Bedeutung sind solche Arzneimittelkombinationen, die die Enantiomere der Formel 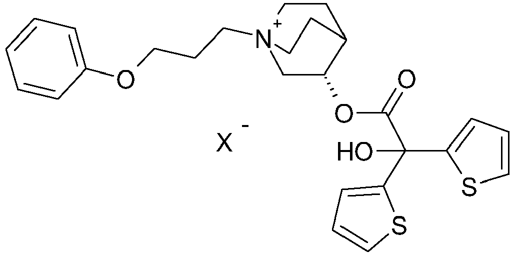 enthalten, worin X ⁻ die vorstehend genannten Bedeutungen aufweisen kann.
Die Konzentration mit der die vorstehend genannten Anticholinergika in den erfindungsgemäßen Arzneimittelzubereitungen enthalten sind liegt erfindungsgemäß bei etwa 4 bis 2000 mg pro 100 g, bevorzugt bei etwa 8 bis 1600 mg pro 100 g. Besonders bevorzugt enthalten 100g der erfindungsgemäßen Formulierungen etwa 80 bis etwa 1360 mg der vorstehend genannten Anticholinergika (bezogen auf pharmakologisch aktives Kation). Werden die vorstehend genannten Bromide eingesetzt, liegt deren Konzentration in den erfindungsgemäßen Zusammensetzungen üblicherweise bei etwa 5 bis 2500 mg pro 100 g, bevorzugt bei etwa 10 bis 2000 mg pro 100 g Arzneimittelzubereitung. Besonders bevorzugt enthalten 100g der erfindungsgemäßen Formulierungen etwa 100 bis 1700 mg eines der vorstehend genannten Bromide.

In einer weiteren bevorzugten Ausführungsform der vorliegenden Erfindung sind die in den erfindungsgemäßen Zubereitungen enthaltenen Anticholinergika ausgewählt aus der Gruppe bestehend aus 2,2-Diphenylpropionsäuretropenolester-methobromid, 2,2-Diphenylpropionsäurescopinester-methobromid, 2-Fluor-2,2-Diphenylessigsäure-scopinester-methobromid, 2-Fluor-2,2-Diphenylessigsäuretropenolester-methobromid, 3,3',4,4'-Tetrafluorbenzilsäuretropenolester-methobromid, 3,3',4,4'-Tetrafluorbenzilsäurescopinester-methobromid, 4,4'-Difluorbenzilsäuretropenolester-methobromid, 4,4'-Difluorbenzilsäurescopinester-methobromid, 3,3'-Difluorbenzilsäuretropenolester-methobromid, 3,3'-Difluorbenzilsäurescopinester-methobromid, 9-Hydroxy-fluoren-9-carbonsäuretropenolester -methobromid, 9-Fluor-fluoren-9-carbonsäuretropenolester - methobromid, 9-Hydroxy-fluoren-9-carbonsäurescopinester -methobromid, 9-Fluor-fluoren-9-carbonsäurescopinester methobromid, 9-Methyl-fluoren-9-carbonsäure-tropenolester methobromid, 9-Methyl-fluoren-9-carbonsäurescopinester methobromid, Benzilsäurecyclopropyltropinester-methobromid, 2,2-Diphenylpropionsäurecyclopropyltropinester-methobromid, 9-Hydroxy-xanthen-9-carbonsäure-cyclopropyltropinester-methobromid, 9-Methyl-fluoren-9-carbonsäure-cyclopropyltropinester-methobromid, 9-Methyl-xanthen-9-carbonsäure-cyclopropyltropinester -methobromid, 9-Hydroxy-fluoren-9-carbonsäure-cyclopropyltropinester -methobromid, 4,4'-Difluorbenzilsäuremethylestercyclopropyltropinester -methobromid, 9-Hydroxy-xanthen-9-carbonsäuretropenolester -methobromid, 9-Hydroxy-xanthen-9-carbonsäurescopinester methobromid, 9-Methyl-xanthen-9-carbonsäuretropenolester -methobromid, 9-Methyl-xanthen-9-carbonsäurescopinester -methobromid, 9-Ethyl-xanthen-9-carbonsäuretropenolester methobromid, 9-Difluormethyl-xanthen-9-carbonsäuretropenolester -methobromid und 9-Hydroxymethyl-xanthen-9-carbonsäurescopinester -methobromid.
Statt der vorstehend genannten Bromide können in den erfindungsgemäßen Zusammensetzungen beispielsweise auch die entsprechenden Fluoride, Chloride, Iodide, Sulfate, Phosphate, Methansulfonate, Nitrate, Maleate, Acetate, Citrate, Fumarate, Tartrate, Oxalate, Succinate, Benzoate oder auch p-Toluolsulfonate enthalten sein, wobei den Bromiden allerdings besondere Bedeutung zukommt.
Die vorstehend genannten Verbindungen können gegebenenfalls in Form ihrer Enantiomere, Mischungen ihrer Enantiomere oder Racemate, sowie gegebenenfalls in Form ihrer Hydrate und/oder Solvate enthalten sein.
Die Konzentration mit der die vorstehend genannten Anticholinergika in den erfindungsgemäßen Arzneimittelzubereitungen enthalten sind liegt erfindungsgemäß bei etwa 4 bis 2000 mg pro 100 g, bevorzugt bei etwa 8 bis 1600 mg pro 100 g. Besonders bevorzugt enthalten 100g der erfindungsgemäßen Formulierungen etwa 80 bis etwa 1360 mg der vorstehend genannten Anticholinergika (bezogen auf pharmakologisch aktives Kation). Werden die vorstehend genannten Bromide eingesetzt, liegt deren Konzentration in den erfindungsgemäßen Zusammensetzungen üblicherweise bei etwa 5 bis 2500 mg pro 100 g, bevorzugt bei etwa 10 bis 2000 mg pro 100 g Arzneimittelzubereitung. Besonders bevorzugt enthalten 100g der erfindungsgemäßen Formulierungen etwa 100 bis 1700 mg eines der vorstehend genannten Bromide.

Als Betamimetika gelangen hierbei vorzugsweise Verbindungen zur Anwendung, die ausgewählt sind aus der Gruppe bestehend aus Albuterol, Arformoterol, Bambuterol, Bitolterol, Broxaterol, Carbuterol, Clenbuterol, Fenoterol, Formoterol, Hexoprenaline, Ibuterol, Isoetharine, Isoprenaline, Levosalbutamol, Mabuterol, Meluadrine, Metaproterenol, Orciprenaline, Pirbuterol, Procaterol, Reproterol, Rimiterol, Ritodrine, Salmefamol, Salmeterol, Soterenol, Sulphonterol, Terbutaline, Tiaramide, Tolubuterol, Zinterol, CHF-1035, HOKU-81, KUL-1248, 3-(4-{6-[2-Hydroxy-2-(4-hydroxy-3-hydroxymethyl-phenyl)-ethylamino]-hexyloxy}-butyl)-benzyl-sulfonamid, 5-[2-(5,6-Diethyl-indan-2-ylamino)-1-hydroxy-ethyl]-8-hydroxy-1H-quinolin-2-on, 4-Hydroxy-7-[2-{[2-{[3-(2-phenylethoxy)propyl]sulphonyl}ethyl]-amino}ethyl]-2(3H)-benzothiazolon, 1-(2-Fluoro-4-hydroxyphenyl)-2-[4-(1-benzimidazolyl)-2-methyl-2-butylamino]ethanol, 1-[3-(4-Methoxybenzyl-amino)-4-hydroxyphenyl]-2-[4-(1-benzimidazolyl)-2-methyl-2-butylamino]ethanol, 1-[2H-5-hydroxy-3-oxo-4H-1,4-benzoxazin-8-yl]-2-[3-(4-N,N-dimethylaminophenyl)-2-methyl-2-propylamino]ethanol, 1-[2H-5-hydroxy-3-oxo-4H-1,4-benzoxazin-8-yl]-2-[3-(4-methoxyphenyl)-2-methyl-2-propylamino]ethanol, 1-[2H-5-hydroxy-3-oxo-4H-1,4-benzoxazin-8-yl]-2-[3-(4-n-butyloxyphenyl)-2-methyl-2-propylamino]ethanol, 1-[2H-5-hydroxy-3-oxo-4H-1,4-benzoxazin-8-yl]-2-{4-[3-(4-methoxyphenyl)-1,2,4-triazol-3-yl]-2-methyl-2-butylamino}ethanol, 5-Hydroxy-8-(1-hydroxy-2-isopropylaminobutyl)-2H-1,4-benzoxazin-3-(4H)-on, 1-(4-Amino-3-chloro-5-trifluormethylphenyl)-2-tert.-butylamino)ethanol, 6-Hydroxy-8-{1-hydroxy-2-[2-(4-methoxy-phenyl)-1,1-dimethyl-ethylamino]-ethyl}-4H-benzo[1,4]oxazin-3-on, 6-Hydroxy-8-{1-hydroxy-2-[2-(4-phenoxy-essigsäureethylester)-1, 1-dimethyl-ethylamino]-ethyl}-4H-benzo[1,4]oxazin-3-on, 6-Hydroxy-8-{1-hydroxy-2-[2-(4-phenoxy-essigsäure)-1,1-dimethyl-ethylamino]-ethyl}-4H-benzo[1,4]oxazin-3-on, 8-{2-[1,1-Dimethyl-2-(2,4,6-trimethylphenyl)-ethylamino]-1-hydroxy-ethyl}-6-hydroxy-4H-benzo[1,4]oxazin-3-on, 6-Hydroxy-8-{1-hydroxy-2-[2-(4-hydroxy-phenyl)-1,1-dimethyl-ethylamino]-ethyl}-4H-benzo[1,4]oxazin-3-on, 6-Hydroxy-8-{1-hydroxy-2-[2-(4-isopropyl-phenyl)-1,1dimethyl-ethylamino]-ethyl}-4H-benzo[,4]oxazin-3-on, 8-{2-[2-(4-Ethyl-phenyl)-1,1-dimethyl-ethylamino]-1-hydroxy-ethyl}-6-hydroxy-4H-benzo[1,4]oxazin-3-on, 8-{2-[2-(4-Ethoxyphenyl)-1,1-dimethyl-ethylamino]-1-hydroxy-ethyl}-6-hydroxy-4H-benzo[1,4]oxazin-3-on, 4-(4-{2-[2-Hydroxy-2-(6-hydroxy-3-oxo-3,4-dihydro-2H-benzo[1,4]oxazin-8-yl)-ethylamino]-2-methyl-propyl}-phenoxy)-buttersäure, 8-{2-[2-(3,4-Difluor-phenyl)-1,1-dimethyl-ethylamino]-1-hydroxy-ethyl}-6-hydroxy-4H-benzo[1,4]oxazin-3-on und 1-(4-Ethoxy-carbonylamino-3-cyano-5-fluorophenyl)-2-(tert.-butylamino)ethanol, gegebenenfalls in Form ihrer Racemate, Enantiomere, Diasteromere und gegebenenfalls in Form ihrer pharmakologisch verträglichen Säureadditionssalze, Solvate oder Hydrate. Erfindungsgemäß bevorzugt sind die Säureadditionssalze der Betamimetika ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Die Konzentration der vorstehend genannten Betamimetika in den erfindungsgemäßen Formulierungen liegt üblicherweise bei etwa 0,1 bis 1600 mg pro 100 g, bevorzugt bei etwa 0,5 bis 1000 mg pro 100 g, besonders bevorzugt 0,75 bis 200 mg pro 100 g. Besonders bevorzugt enthalten 100g der erfindungsgemäßen Formulierungen etwa 1 bis etwa 100 mg der vorstehend genannten Betamimetika (jeweils bezogen auf die freie Base der vorstehend genannten Verbindungen).

Als Corticosteroide gelangen hierbei vorzugsweise Verbindungen zur Anwendung, die ausgewählt sind aus der Gruppe bestehend aus Prednisolon, Prednison, Butixocortpropionat, Flunisolid, Beclomethason, Triamcinolon, Budesonid, Fluticason, Mometason, Ciclesonid, Rofleponid, Dexamethason, Betamethason, Deflazacort, RPR-106541, NS-126, ST-26, 6,9-Difluoro-17-[(2-furanylcarbonyl)oxy]-11-hydroxy-16-methyl-3-oxo-androsta-1,4-dien-17-carbothionsäure (S)-fluoromethylester, 6,9-Difluoro-11-hydroxy-16-methyl-3-oxo-17-propionyloxy-androsta-1,4-dien-17-carbothionsäure (S)-(2-oxo-tetrahydro-furan-3S-yl)ester und
Etiprednol-dichloroacetat gegebenenfalls in Form ihrer Racemate, Enantiomere oder Diastereomere und gegebenenfalls in Form ihrer Salze und Derivate, ihrer Solvate und/oder Hydrate. Jede Bezugnahme auf Steroide schließt eine Bezugnahme auf deren gegebenenfalls existierende Salze oder Derivate, Hydrate oder Solvate mit ein. Beispiele möglicher Salze und Derivate der Steroide können sein: Alkalisalze, wie beispielsweise Natrium- oder Kaliumsalze, Sulfobenzoate, Phosphate, Isonicotinate, Acetate, Propionate, Dihydrogenphosphate, Palmitate, Pivalate oder auch Furoate.
Die Konzentration der vorstehend genannten Steroide in den erfindungsgemäßen Formulierungen liegt üblicherweise bei etwa 10 bis 1800 mg pro 100 g, bevorzugt bei etwa 100 bis 1500 mg pro 100 g, besonders bevorzugt 200 bis 1000 mg pro 100 g. Besonders bevorzugt enthalten 100g der erfindungsgemäßen Formulierungen etwa 400 bis etwa 700 mg der vorstehend genannten Steroide.

Als PDE4-Inhibitoren gelangen hierbei vorzugsweise Verbindungen zur Anwendung, die ausgewählt sind aus der Gruppe bestehend aus Enprofyllin, Theophyllin, Roflumilast, Ariflo (Cilomilast), Tofimilast, Pumafentrin, Lirimilast, Arofyllin, Atizoram, D-4418, Bay-198004, BY343, CP-325,366, D-4396 (Sch-351591), AWD-12-281 (GW-842470), NCS-613, CDP-840, D-4418, PD-168787, T-440, T-2585, V-11294A, Cl-1018, CDC-801, CDC-3052, D-22888, YM-58997, Z-15370, N-(3,5-Dichloro-1-oxo-pyridin-4-yl)-4-difluoromethoxy-3-cyclopropylmethoxybenzamid, (-)p-[(4aR*,10*b*S*)-9-Ethoxy-1,2,3,4,4a,10b-hexahydro-8-methoxy-2-methylbenzo[s][1,6]naphthyridin-6-yl]-N,N-diisopropylbenzamid, (R)-(+)-1-(4-Bromobenzyl)-4-[(3-cyclopentyloxy)-4-methoxyphenyl]-2-pyrrolidon, 3-(Cyclopentyloxy-4-methoxyphenyl)-1-(4-N'-[N-2-cyano-S-methyl-isothioureido]benzyl)-2-pyrrolidon, cis[4-Cyano-4-(3-cyclopentyloxy-4-methoxyphenyl)cyclohexan-1-carbonsäure], 2-carbomethoxy-4-cyano-4-(3-cyclopropylmethoxy-4-difluoromethoxyphenyl)cyclohexan-1-on, cis[4-Cyano-4-(3-cyclopropylmethoxy-4-difluoromethoxyphenyl)cyclohexan-1-ol], (R)-(+)-Ethyl[4-(3-cyclopentyloxy-4-methoxyphenyl)pyrrolidin-2-yliden]acetat, (S)-(-)-Ethyl[4-(3-cyclopentyloxy-4-methoxyphenyl)pyrrolidin-2-yliden]acetat, 9-Cyclopentyl-5,6-dihydro-7-ethyl-3-(2-thienyl)-9*H*-pyrazolo[3,4-c]-1,2,4-triazolo[4,3-a]pyridin und 9-Cyclopentyl-5,6-dihydro-7-ethyl-3-(*tert*-butyl)-9*H*-pyrazolo[3,4-c]-1,2,4-triazolo[4,3-a]pyridin, gegebenenfalls in Form ihrer Racemate, Enantiomere, Diasteromere und gegebenenfalls in Form ihrer pharmakologisch verträglichen Säureadditionssalze, Solvate oder Hydrate. Erfindungsgemäß bevorzugt sind die Säureadditionssalze der PDE4-Inhibitoren ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.
Die Konzentration der vorstehend genannten PDE4-Inhibitoren in den erfindungsgemäßen Formulierungen liegt üblicherweise bei etwa 1 bis 1500 mg pro 100 g, bevorzugt bei etwa 10 bis 1200 mg pro 100 g, besonders bevorzugt 100 bis 1000 mg pro 100 g. Besonders bevorzugt enthalten 100g der erfindungsgemäßen Formulierungen etwa 150 bis etwa 800 mg der vorstehend genannten PDE4-Inhibitoren (jeweils bezogen auf die freie Base der vorstehend genannten Verbindungen).

Als LTD4-Antagonisten gelangen hierbei vorzugsweise Verbindungen zur Anwendung, die ausgewählt sind aus der Gruppe bestehend aus Montelukast, Pranlukast, Zafirlukast, MCC-847 (ZD-3523), MN-001, MEN-91507 (LM-1507), VUF-5078, VUF-K-8707, L-733321, 1-(((R)-(3-(2-(6,7-Difluoro-2-quinolinyl)ethenyl)phenyl)-3-(2-(2-hydroxy-2-propyl)phenyl)thio)methylcyclopropan-essigsäure, 1-(((1(R)-3(3-(2-(2,3-Dichloro-thieno[3,2-b]pyridin-5-yl)-(E)-ethenyl)phenyl)-3-(2-(1-hydroxy-1-methylethyl)phenyl)-propyl)thio)methyl)cyclopropanessigsäure und [2-[[2-(4-tert-Butyl-2-thiazolyl)-5-benzofuranyl]oxymethyl]phenyl]essigsäure gegebenenfalls in Form ihrer Racemate, Enantiomere, Diasteromere und gegebenenfalls in Form ihrer pharmakologisch verträglichen Säureadditionssalze, Solvate oder Hydrate. Erfindungsgemäß bevorzugt sind die Säureadditionssalze der LTD4-Antagonisten ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat. Unter Salzen oder Derivaten zu deren Bildung die LTD4-antagonisten gegebenenfalls in der Lage sind, werden beispielsweise verstanden: Alkalisalze, wie beispielsweise Natrium- oder Kaliumsalze, Erdalkalisalze, Sulfobenzoate, Phosphate, Isonicotinate, Acetate, Propionate, Dihydrogenphosphate, Palmitate, Pivalate oder auch Furoate.
Die Konzentration der vorstehend genannten LTD4-antagonisten in den erfindungsgemäßen Formulierungen liegt üblicherweise bei etwa 0,1 bis 1600 mg pro 100 g, bevorzugt bei etwa 0,5 bis 1000 mg pro 100 g, besonders bevorzugt 0,75 bis 200 mg pro 100 g.

Als Dopamin-Agonisten gelangen hierbei vorzugsweise Verbindungen zur Anwendung, die ausgewählt sind aus der Gruppe bestehend aus Bromocriptin, Cabergolin, Alpha-Dihydroergocryptin, Lisurid, Pergolid, Pramipexol, Roxindol, Ropinirol, Talipexol, Tergurid und Viozan, gegebenenfalls in Form ihrer Racemate, Enantiomere, Diasteromere und gegebenenfalls in Form ihrer pharmakologisch verträglichen Säureadditionssalze, Solvate oder Hydrate. Erfindungsgemäß bevorzugt sind die Säureadditionssalze der Dopamin-Agonisten ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.
Die Konzentration der vorstehend genannten Dopamin-agonisten in den erfindungsgemäßen Formulierungen liegt üblicherweise bei etwa 0,1 bis 1500 mg pro 100 g, bevorzugt bei etwa 1 bis 1000 mg pro 100 g, besonders bevorzugt 5 bis 750 mg pro 100 g (jeweils bezogen auf die freie Base der vorstehend genannten Verbindungen).

Als H1-Antihistaminika gelangen hierbei vorzugsweise Verbindungen zur Anwendung, die ausgewählt sind aus der Gruppe bestehend aus Epinastin, Cetirizin, Azelastin, Fexofenadin, Levocabastin, Loratadin, Mizolastin, Ketotifen, Emedastin, Dimetinden, Clemastin, Bamipin, Cexchlorpheniramin, Pheniramin, Doxylamin, Chlorphenoxamin, Dimenhydrinat, Diphenhydramin, Promethazin, Ebastin, Desloratidin und Meclozin, gegebenenfalls in Form ihrer Racemate, Enantiomere, Diasteromere und gegebenenfalls in Form ihrer pharmakologisch verträglichen Säureadditionssalze, Solvate oder Hydrate.

Erfindungsgemäß bevorzugt sind die Säureadditionssalze der H1-Antihistaminika ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.
Die Konzentration der vorstehend genannten H1-Antihistaminika in den erfindungsgemäßen Formulierungen liegt üblicherweise bei etwa 1 bis 1500 mg pro 100 g, bevorzugt bei etwa 10 bis 1000 mg pro 100 g, besonders bevorzugt 20 bis 800 mg pro 100 g (jeweils bezogen auf die freie Base der vorstehend genannten Verbindungen).

Ein weiterer Aspekt der vorliegenden Erfindung betrifft die Verwendung der erfindungsgemäßen Arzneimittelformulierungen zur Herstellung eines Arzneimittels zur Behandlung von Atemwegserkrankungen, die ausgewählt sind aus der Gruppe bestehend aus Obstruktive Lungenerkrankungen unterschiedlicher Genese, Lungenemphyseme unterschiedlicher Genese, Restriktive Lungenerkrankungen, Interstitielle Lungenerkrankungen, Zystische Fibrose, Bronchitiden unterschiedlicher Genese, Bronchiektasen, ARDS (adult respiratory distress syndrom) und alle Formen des Lungenödems.

Bevorzugt ist die vorstehend genannte Verwendung zur Herstellung eines Arzneimittels zur Behandlung von Obstruktive Lungenerkrankungen die ausgewählt sind aus der Gruppe bestehend aus Asthma Bronchiale, pädiatrisches Asthma, schweres Asthma, akuter Asthma-Anfall, chronische Bronchitis und chronisch obstruktive Lungenerkrankung (COPD), wobei die Verwendung zur Herstellung eines Arzneimittels zur Behandlung von Asthma Bronchiale oder COPD erfindungsgemäß besonders bevorzugt ist.

Bevorzugt ist ferner die Verwendung der erfindungsgemäßen Arzneimittelformulierungen zur Herstellung eines Arzneimittels zur Behandlung von Lungenemphysemen die ihren Ursprung haben in COPD (chronisch obstruktive pulmonale Erkrankung) oder α1-Proteinase-Inhibitor-Mangel.

Bevorzugt ist ferner die Verwendung der erfindungsgemäßen Arzneimittelformulierungen zur Herstellung eines Arzneimittels zur Behandlung von Restriktiven Lungenerkrankungen, die ausgewählt sind aus der Gruppe bestehend aus Allergische Alveolitis, durch berufliche Noxen ausgelöste restriktive Lungenerkrankungen wie Asbestose oder Silikose und Restriktion aufgrund von Lungentumoren, wie beispielsweise Lymphangiosis carcinomatosa, brochoalveoläres Karzinom und Lymphome.

Bevorzugt ist ferner die Verwendung der erfindungsgemäßen Arzneimittelformulierungen zur Herstellung eines Arzneimittels zur Behandlung von Interstitiellen Lungenerkrankungen, die ausgewählt sind aus der Gruppe bestehend aus infektiös bedingte Pneumonien, wie beispielsweise aufgrund einer Infektion mit Viren, Bakterien, Pilzen, Protozoen, Helminthen oder anderen Erregern, Pneumonitis aufgrund unterschiedlicher Genese, wie beispielsweise Aspiration und Linksherzinsuffizienz, Strahlen-induzierte Pneumonitis oder Fibrose, Kollagenosen, wie beispielsweise Lupus erythematodes, sytemische Sklerodermie oder Sarkoidose, Granulomatosen, wie beispielsweise Morbus Boeck, idiopathische interstitielle Pneumonie oder idiopathische pulmonäre Fibrose (IPF).

Bevorzugt ist ferner die Verwendung der erfindungsgemäßen Arzneimittelformulierungen zur Herstellung eines Arzneimittels zur Behandlung von Zystischer Fibrose bzw. Mukoviszidose.

Bevorzugt ist ferner die Verwendung der erfindungsgemäßen Arzneimittelformulierungen zur Herstellung eines Arzneimittels zur Behandlung von Bronchitiden, wie beispielsweise Bronchitis aufgrund bakterieller oder viraler Infektion, Allergische Bronchitis und Toxische Bronchitis.

Bevorzugt ist ferner die Verwendung der erfindungsgemäßen Arzneimittelformulierungen zur Herstellung eines Arzneimittels zur Behandlung von Bronchiektasen.

Bevorzugt ist ferner die Verwendung der erfindungsgemäßen Arzneimittelformulierungen zur Herstellung eines Arzneimittels zur Behandlung von ARDS (adult respiratory distress syndrom).

Bevorzugt ist ferner die Verwendung der erfindungsgemäßen Arzneimittelformulierungen zur Herstellung eines Arzneimittels zur Behandlung von Lungenödemen, beispielsweise toxischer Lungenödeme nach Aspiration oder Inhalation von toxischen Substanzen und Fremdstoffen.

Besonders bevorzugt betrifft die vorliegende Erfindung die Verwendung der erfindungsgemäßen Arzneimittelformulierungen zur Herstellung eines Arzneimittels zur Behandlung von Asthma oder COPD. Von besonderer Bedeutung ist ferner die vorstehend genannte Verwendung zur Herstellung eines Arzneimittels zur einmal täglichen Behandlung von entzündlichen und obstruktiven Atemwegserkrankungen, besonders bevorzugt zur einmal täglichen Behandlung von Asthma oder COPD.

Ferner betrifft die vorliegende Erfindung ein Verfahren zur Behandlung der vorstehend genannten Erkrankungen, dadurch gekennzeichnet, daß eine oder mehrere der vorstehend genannten erfindungsgemäßen Arzneimittelformulierungen in therapeutisch wirksamen Mengen appliziert werden.

Die vorliegende Erfindung beschäftigt sich mit inhalativ applizierbaren flüssigen Wirkstoffformulierungen dieser Verbindungen, wobei die erfindungsgemäßen flüssigen Formulierungen hohen Qualitätsstandards genügen müssen. Die erfindungsgemäßen Formulierungen können dabei peroral oder pernasal inhaliert werden. Um eine optimale Wirkstoffverteilung der Wirksubstanzen in der Lunge zu erhalten, bietet sich die Applikation einer flüssigen, auf Treibgase verzichtenden, Formulierung mittels dafür geeigneter Inhalatoren an. Die inhalative Applikation einer solchen Formulierung kann sowohl auf oralem als auch auf nasalem Weg erfolgen. Besonders geeignet sind solche Inhalatoren, die eine kleine Menge einer flüssigen Formulierung in der therapeutisch notwendigen Dosierung binnen weniger Sekunden in ein therapeutisch-inhalativ geeignetes Aerosol vernebeln können. Im Rahmen der vorliegenden Erfindung sind solche Vernebler bevorzugt, bei denen bereits eine Menge von weniger als 100 Mikrolitern, bevorzugt weniger als 50 Mikrolitern, ganz bevorzugt weniger als 25 Mikrolitern Wirkstofflösung mit bevorzugt einem Hub oder zwei Hüben zu einem Aerosol mit einer durchschnittlichen Teilchengröße (bzw. Teilchendurchmesser) von weniger als 20 Mikrometern, bevorzugt weniger als 10 Mikrometern, so vernebelt werden können, dass der inhalierbare Anteil des Aerosols bereits der therapeutisch wirksamen Menge entspricht.
Eine derartige Vorrichtung zur treibgasfreien Verabreichung einer dosierten Menge eines flüssigen Arzneimittels zur inhalativen Anwendung, wird beispielsweise in der internationalen Patentanmeldung WO 91/14468 "Atomizing Device and Methods" als auch in der WO 97/12687, dort Figuren 6a und 6b und der dazugehörigen Beschreibung, ausführlich beschrieben. In einem solchen Vernebler wird eine Arzneimittellösung mittels hohen Drucks von bis zu 500 bar in ein lungengängiges Aerosol überführt und versprüht.

Auf die genannten Referenzen wird im Rahmen der vorliegenden Erfindungsbeschreibung ausdrücklich in Gänze Bezug genommen.
In solchen Inhalatoren werden die Lösungsformulierungen in einem Reservoir gelagert. Dabei ist es notwendig, dass die verwendeten Wirkstoffformulierungen eine ausreichende Lagerstabilität aufweisen und gleichzeitig so beschaffen sind, dass sie dem medizinischen Zweck entsprechend möglichst ohne weitere Manipulation, direkt appliziert werden können. Ferner dürfen sie keine Bestandteile aufweisen, die so mit dem Inhalator wechselwirken können, dass der Inhalator oder die pharmazeutische Qualität der Lösung, respektive des erzeugten Aerosols, Schaden nehmen könnte.

Zur Vernebelung der Lösung wird eine spezielle Düse verwendet, wie sie beispielsweise die WO 94/07607 oder die WO 99/16530 beschreibt. Auf beide wird hiermit ausdrücklich Bezug genommen.

Es ist Aufgabe der vorliegenden Erfindung, eine wässrige, ethanolische oder wässerigethanolische Formulierung der Verbindung der Formel **1** bereitzustellen, welche den hohen Standards genügt, die notwendig sind, um eine Lösung mittels der eingangs genannten Inhalatoren optimal vernebeln zu können. Die erfindungsgemäßen Wirkstoffformulierungen müssen dabei auch eine ausreichende pharmazeutische Qualität aufweisen, d.h. sie sollten über eine Lagerzeit von einigen Jahren, bevorzugt von mindestens einem Jahr, stärker bevorzugt von zwei Jahren pharmazeutisch stabil sein. Diese treibgasfreien Lösungsformulierungen müssen ferner mittels eines Inhalators unter Druck vernebelt werden können, wobei die im generierten Aerosol ausgebrachte Masse reproduzierbar innerhalb eines definierten Bereichs liegt.

Die erfindungsgemäßen Arzneimittelformulierungen werden bevorzugt in einem Inhalator der vorstehend beschriebenen Art verwendet, um daraus die erfindungsgemäßen treibgasfreien Aerosole herzustellen. An dieser Stelle sei deshalb noch einmal ausdrücklich auf die eingangs beschriebenen Patentdokumente verwiesen, auf die hiermit vollinhaltlich Bezug genommen wird.

Wie eingangs geschildert wird eine weiterentwickelte Ausführungsform des bevorzugten Inhalators in der WO 97/12687 (siehe dort insbesondere Figuren 6a und 6b und die diesbezüglichen Beschreibungsteile) offenbart. Dieser Vernebler (Respimat®) kann vorteilhaft zur Erzeugung der erfindungsgemäßen inhalierbaren Aerosole eingesetzt werden. Aufgrund seiner zylinderähnlichen Form und einer handlichen Größe von weniger als 9 bis 15 cm in der Länge und 2 bis 4 cm in der Breite kann dieses Device jederzeit vom Patienten mitgeführt werden. Der Vernebler versprüht ein definiertes Volumen der Arzneimittelformulierung unter Anwendung hoher Drücke durch kleine Düsen, so dass inhalierbare Aerosole entstehen.

Im wesentlichen besteht der bevorzugte Zerstäuber aus einem Gehäuseoberteil, einem Pumpengehäuse, einer Düse, einem Sperrspannwerk, einem Federgehäuse, einer Feder und einem Vorratsbehälter, gekennzeichnet durch
- ein Pumpengehäuse, das im Gehäuseoberteil befestigt ist, und das an seinem einen Ende einen Düsenkörper mit der Düse bzw. Düsenanordnung trägt,
- einen Hohlkolben mit Ventilkörper,
- einen Abtriebsflansch, in dem der Hohlkolben befestigt ist, und der sich im Gehäuseoberteil befindet,
- ein Sperrspannwerk, das sich im Gehäuseoberteil befindet,
- ein Federgehäuse mit der darin befindlichen Feder, das am Gehäuseoberteil mittels eines Drehlagers drehbar gelagert ist,
- ein Gehäuseunterteil, das auf das Federgehäuse in axialer Richtung aufgesteckt ist.

Der Hohlkolben mit Ventilkörper entspricht einer in der WO 97/12687 offenbarten Vorrichtungen. Er ragt teilweise in den Zylinder des Pumpengehäuses hinein und ist im Zylinder axial verschiebbar angeordnet. Insbesondere wird auf die Figuren 1-4 - insbesondere Figur 3 - und die dazugehörigen Beschreibungsteile der o.g. Internationalen Patentanmeldung Bezug genommen. Der Hohlkolben mit Ventilkörper übt auf seiner Hochdruckseite zum Zeitpunkt des Auslösens der Feder einen Druck von 5 bis 60 MPa (etwa 50 bis 600 bar), bevorzugt 10 bis 60 MPa (etwa 100 bis 600 bar) auf das Fluid, die abgemessene Wirkstofflösung aus. Dabei werden Volumina von 10 bis 50 Mikroliter bevorzugt, besonders bevorzugt sind Volumina von 10 bis 20 Mikroliter, ganz besonders bevorzugt ist ein Volumen von 10 bis 15 Mikroliter pro Hub.

Der Ventilkörper ist bevorzugt an dem Ende des Hohlkolbens angebracht, das dem Düsenkörper zugewandt ist.

Die Düse im Düsenkörper ist bevorzugt mikrostrukturiert, d.h. durch Mikrotechnik hergestellt. Mikrostrukturierte Düsenkörper sind beispielsweise in der WO-99/16530 offenbart; auf diese Schrift wird hiermit inhaltlich Bezug genommen, insbesondere auf die dort offenbarte Figur 1 und deren Beschreibung.
Der Düsenkörper besteht z.B. aus zwei fest miteinander verbundenen Platten aus Glas und/oder Silizium, von denen wenigstens eine Platte einen oder mehrere mikrostrukturierte Kanäle aufweist, die die Düseneinlaßseite mit der Düsenauslaßseite verbinden. Auf der Düsenauslaßseite ist mindestens eine runde oder nicht-runde Öffnung von 2 bis 10 Mikrometer Tiefe und 5 bis 15 Mikrometern Breite, wobei die Tiefe bevorzugt bei 4, 5 bis 6,5 Mikrometern und die Länge bei 7 bis 9 Mikrometern beträgt.
Im Fall von mehreren Düsenöffnungen, bevorzugt sind zwei, können die Strahlrichtungen der Düsen im Düsenkörper parallel zueinander verlaufen oder sie sind in Richtung Düsenöffnung gegeneinander geneigt. Bei einem Düsenkörper mit mindestens zwei Düsenöffnungen auf der Auslaßseite können die Strahlrichtungen mit einem Winkel von 20 Grad bis 160 Grad gegeneinander geneigt sein, bevorzugt wird ein Winkel von 60 bis 150 Grad, insbesondere bevorzugt 80 bis 100°.
Die Düsenöffnungen sind bevorzugt in einer Entfernung von 10 bis 200 Mikrometern angeordnet, stärker bevorzugt in einer Entfernung von 10 bis 100 Mikrometer, besonders bevorzugt 30 bis 70 Mikrometer. Am stärksten bevorzugt sind 50 Mikrometer.
Die Strahlrichtungen treffen sich dementsprechend in der Umgebung der Düsenöffnungen.

Die flüssige Arzneimittelformulierung trifft wie bereits erwähnt mit einem Eingangsdruck von bis zu 600 bar, bevorzugt 200 bis 300 bar auf den Düsenkörper und wird über die Düsenöffnungen in ein inhalierbares Aerosol zerstäubt. Die bevorzugten Teilchengrößen des Aerosols liegen bei bis zu 20 Mikrometern, bevorzugt 3 bis 10 Mikrometern.

Das Sperrspannwerk enthält eine Feder, bevorzugt eine zylindrische schraubenförmige Druckfeder, als Speicher für die mechanische Energie. Die Feder wirkt auf den Abtriebsflansch als Sprungstück, dessen Bewegung durch die Position eines Sperrglieds bestimmt wird. Der Weg des Abtriebsflansches wird durch einen oberen und einen unteren Anschlag präzise begrenzt. Die Feder wird bevorzugt über ein kraftübersetzendes Getriebe, z.B. ein Schraubschubgetriebe, durch ein äußeres Drehmoment gespannt, das beim Drehen des Gehäuseoberteils gegen das Federgehäuse im Gehäuseunterteil erzeugt wird. In diesem Fall enthalten das Gehäuseoberteil und der Abtriebsflansch ein ein- oder mehrgängiges Keilgetriebe.

Das Sperrglied mit einrückenden Sperrflächen ist ringförmig um den Abtriebsflansch angeordnet. Es besteht z.B. aus einem in sich radial elastisch verformbaren Ring aus Kunststoff oder aus Metall. Der Ring ist in einer Ebene senkrecht zur Zerstäuberachse angeordnet. Nach dem Spannen der Feder schieben sich die Sperrflächen des Sperrgliedes in den Weg des Abtriebsflansches und verhindern das Entspannen der Feder. Das Sprerrglied wird mittels einer Taste ausgelöst. Die Auslösetaste ist mit dem Sperrglied verbunden oder gekoppelt. Zum Auslösen des Sperrspannwerkes wird die Auslösetaste parallel zur Ringebene, und zwar bevorzugt in den Zerstäuber hinein, verschoben; dabei wird der verformbare Ring in der Ringebene verformt. Konstruktive Details des Sperrspannwerkes sind in der WO 97/20590 beschrieben.

Das Gehäuseunterteil wird in axialer Richtung über das Federgehäuse geschoben und verdeckt die Lagerung, den Antrieb der Spindel und den Vorratsbehälter für das Fluid.

Beim Betätigen des Zerstäubers wird das Gehäuseoberteil gegen das Gehäuseunterteil gedreht, wobei das Gehäuseunterteil das Federgehäuse mitnimmt. Dabei wird die Feder über das Schraubschubgetriebe zusammengedrückt und gespannt, und das Sperrwerk rastet selbsttätig ein. Der Drehwinkel ist bevorzugt ein ganzzahliger Bruchteil von 360 Grad, z.B. 180 Grad. Gleichzeitig mit dem Spannen der Feder wird das Abtriebsteil im Gehäuseoberteil um einen vorgegebenen Weg verschoben, der Hohlkolben wird innerhalb des Zylinders im Pumpengehäuse zurückgezogen, wodurch eine Teilmenge des Fluids aus dem Vorratsbehälter in den Hochdruckraum vor der Düse eingesaugt wird.

In den Zerstäuber können gegebenenfalls nacheinander mehrere das zu zerstäubende Fluid enthaltende austauschbare Vorratsbehälter eingeschoben und benutzt werden. Der Vorratsbehälter enthält die erfindungsgemäße Aerosolzubereitung.

Der Zerstäubungsvorgang wird durch leichtes Eindrücken der Auslösetaste eingeleitet. Dabei gibt das Sperrwerk den Weg für das Abtriebsteil frei. Die gespannte Feder schiebt den Kolben in den Zylinder des Pumpengehäuses hinein. Das Fluid tritt aus der Düse des Zerstäubers in zerstäubter Form aus.

Weitere konstruktive Details sind in den PCT-Anmeldungen WO 97/12683 und WO 97/20590 offenbart, auf die hiermit inhaltlich Bezug genommen wird.

Die Bauteile des Zerstäubers (Verneblers) sind aus einem der Funktion entsprechend geeignetem Material. Das Gehäuse des Zerstäubers und - so weit es die Funktion erlaubt - auch andere Teile sind bevorzugt aus Kunststoff, z.B. im Spritzgießverfahren, hergestellt. Für medizinische Zwecke werden physiologisch unbedenkliche Materialien verwendet.

In den Figuren 6 a/b der WO 97/12687, ist der Vernebler (Respimat®) beschrieben, mit dem die erfindungsgemäßen wäßrigen Aerosolzubereitungen vorteilhaft inhaliert werden können. Figur 6 a zeigt einen Längsschnitt durch den Zerstäuber bei gespannter Feder, Figur 6 b zeigt einen Längsschnitt durch den Zerstäuber bei entspannter Feder.

Das Gehäuseoberteil (51) enthält das Pumpengehäuse (52), an dessen Ende der Halter (53) für die Zerstäuberdüse angebracht ist. In dem Halter befindet sich der Düsenkörper (54) und ein Filter (55). Der im Abtriebsflansch (56) des Sperrspannwerkes befestigte Hohlkolben (57) ragt teilweise in den Zylinder des Pumpengehäuses hinein. An seinem Ende trägt der Hohlkolben den Ventilkörper (58). Der Hohlkolben ist mittels der Dichtung (59) abgedichtet. Innerhalb des Gehäuseoberteils befindet sich der Anschlag (60), an dem der Abtriebsflansch bei entspannter Feder anliegt. Am Abtriebsflansch befindet sich der Anschlag (61), an dem der Abtriebsflansch bei gespannter Feder anliegt. Nach dem Spannen der Feder schiebt sich das Sperrglied (62) zwischen den Anschlag (61) und eine Abstützung (63) im Gehäuseoberteil. Die Auslösetaste (64) steht mit dem Sperrglied in Verbindung. Das Gehäuseoberteil endet im Mundstück (65) und ist mit der aufsteckbaren Schutzkappe (66) verschlossen.

Das Federgehäuse (67) mit Druckfeder (68) ist mittels der Schnappnasen (69) und Drehlager am Gehäuseoberteil drehbar gelagert. Über das Federgehäuse ist das Gehäuseunterteil (70) geschoben. Innerhalb des Federgehäuses befindet sich der austauschbare Vorratsbehälter (71) für das zu zerstäubende Fluid (72). Der Vorratsbehälter ist mit dem Stopfen (73) verschlossen, durch den der Hohlkolben in den Vorratsbehälter hineinragt und mit seinem Ende in das Fluid (Vorrat an Wirkstofflösung) eintaucht.

In der Mantelfläche des Federgehäuses ist die Spindel (74) für das mechanische Zählwerk angebracht. An dem Ende der Spindel, das dem Gehäuseoberteil zugewandt ist, befindet das Antriebsritzel (75). Auf der Spindel sitzt der Reiter (76).

Der oben beschriebene Vernebler ist geeignet, die erfindungsgemäßen Aerosolzubereitungen zu einem für die Inhalation geeignetem Aerosol zu vernebeln.

Wird die erfindungsgemäße Formulierung mittels der vorstehend beschriebenen Technik (Respimat®) vernebelt, sollte die ausgebrachte Masse bei wenigstens 97%, bevorzugt wenigstens 98% aller Betätigungen des Inhalators (Hub oder Hübe) einer definierten Menge mit einem Toleranzbereichs von maximal 25%, bevorzugt 20% dieser Menge entsprechen. Bevorzugt werden pro Hub zwischen 5 und 30 mg Formulierung als definierte Masse ausgebracht, besonders bevorzugt zwischen 5 und 20 mg.

Die erfindungsgemäße Formulierung kann auch mittels anderer als der vorstehend beschriebenen Inhalatoren, beispielsweise Jet-Stream-Inhalatoren, vernebelt werden. Nachstehend sind Bespiele für Geräte aufgeführt, in denen die erfindungsgemäßen Formulierungen zur Anwendung gelangen können. Es sind dies beispielsweise Geräte gemäß den internationalen Patentanmeldungen WO 02/51466, WO 03/49792 und WO 04/22242 (Chrysalis), Geräte gemäß den internationalen Patentanmeldungen WO 94/14543, WO 00/35524, WO 00/38770 und WO 00/64590 (Battelle / Ventaira), Geräte gemäß den Veröffentlichungen US 20060048772, US 20050224076 und WO 05/42075 (Pari), Geräte gemäß den Veröffentlichungen WO 94/16755, WO 94/16717, WO 96/13291, WO 96/13161, WO 98/22169, WO 98/33480, WO 98/48878 und WO 02/74375 (Aradigm) sowie Geräte gemäß der Veröffentlichung EP 1211628 (Canon).

Die vorliegende Erfindung betrifft ferner ein Inhalationskit bestehend aus einer der vorstehend beschriebenen erfindungsgemäßen Arzneimittelzubereitungen und einem zur Vernebelung dieser Arzneimittelformulierung geeigneten Inhalator.
Die vorliegende Erfindung betrifft bevorzugt ein Inhalationskit bestehend aus einer der vorstehend beschriebenen erfindungsgemäßen Arzneimittelzubereitungen und dem vorstehend beschriebenen Inhalator Respimat®.

Die nachstehend ausgeführten Formulierungsbeispiele dienen der weitergehenden Erläuterung ohne den Gegenstand der vorliegenden Erfindung auf die exemplarisch dargestellten Zusammensetzungen zu beschränken.

In den nachstehenden Formulierungsbeispielen steht BAC für Benzalkoniumchlorid und EDTA für Dinatriumedetat-Dihydrat. Bei den prozentualen Angaben handelt es sich um Gewichtsprozent, wobei der Ethanolanteil allerdings in Volumenprozent angegeben wird.

### A) Tiotropiumbromid-Formulierungen

100 g Arzneimittelformulierung (pH jeweils mit HCl eingestellt auf 2,9±0,2) enthalten in gereinigtem Wasser bzw. Wasser für Injektionszwecke:

| Beispiel | Ethanol (Vol. %) | BAC (mg) | EDTA (mg) | Hilfsstoff (%) | Tiotropium* (%) |
|---|---|---|---|---|---|
| 1 | 0 | 10 | 50 | NaCl (10) | 0,1 |
| 2 | 0 | 10 | 50 | NaCl (15) | 0,2 |
| 3 | 0 | 5 | 30 | Lactose (5) | 0,2 |
| 4 | 0 | 5 | 30 | KCl (10) | 0,2 |
| 5 | 0 | 5 | 20 | NaCl (10) | 0,2 |
| 6 | 0 | 5 | 40 | KCl (10) | 0,1 |
| 7 | 20 | 10 | 30 | Mannitol (10) | 0,2 |
| 8 | 40 | 5 | 50 | NaCl (5) | 0,2 |
| 9 | 0 | 10 | 10 | NaCl (10) | 0,023 |
| 10 | 0 | 10 | 10 | NaCl (15) | 0,045 |

| | | | | | |
|---|---|---|---|---|---|
| * bezogen auf freies Kation | | | | | |

### B) Ipratropiumbromid-Formulierungen

100 g Arzneimittelformulierung (pH jeweils mit HCl eingestellt auf 3,2±0,4) enthalten in gereinigtem Wasser bzw. Wasser für Injektionszwecke:

| Beispiel | Ethanol (Vol. %) | BAC (mg) | EDTA (mg) | Hilfsstoff (%) | Ipratropium* (%) |
|---|---|---|---|---|---|
| 1 | 0 | 5 | 40 | NaCitrat (10) | 1,0 |
| 2 | 0 | 5 | 40 | Lactose (5) | 1,0 |
| 3 | 80 | 5 | 20 | Sorbitol (10) | 1,0 |
| 4 | 95 | 5 | 20 | Sorbitol (4) | 1,0 |
| 5 | 0 | 10 | 50 | Na₃PO₄ (10) | 1,0 |
| 6 | 0 | 10 | 50 | NaHCO₃ (15) | 1,0 |
| 7 | 0 | 10 | 50 | KCl (10) | 1,0 |

| | | | | | |
|---|---|---|---|---|---|
| * bezogen auf Ipratropiumbromid-Monohydrat | | | | | |

### C) Fenoterol-Formulierungen

100 g Arzneimittelformulierung (pH jeweils mit HCl eingestellt auf 3,2±0,4) enthalten in gereinigtem Wasser bzw. Wasser für Injektionszwecke:

| Beispiel | Ethanol (Vol. %) | BAC (mg) | EDTA (mg) | Hilfsstoff (%) | Fenoterol* (%) |
|---|---|---|---|---|---|
| 1 | 60 | 5 | 30 | Sorbitol (15) | 1,0 |
| 2 | 0 | 5 | 10 | Glucose (10) | 1,0 |
| 3 | 0 | 10 | 50 | Glucose (10) | 1,0 |

| | | | | | |
|---|---|---|---|---|---|
| * bezogen auf Hydrobromid | | | | | |

### Bestimmung der Tröpfchengrößen

Die Bestimmung der Teilchengrößenverteilung bzw. des mittleren Volumendurchmessers erfolgt mittels Laserbeugung, Partikelgrößenmessgerät Fa. Sympatec, Modell Helos BF, bei einem Volumenstrom von 28,31/min und den Klimabedingungen von ca. 23°C / ca. 100% relativer Luftfeuchte.

Zur Erzeugung der ca. 100% relativen Luftfeuchte wird eine Druckluftquelle mit einer Befeuchtungsapparatur, die mit Wasser befüllt ist, verbunden. Die Druckluft strömt durch die mit Wasser gefüllte Befeuchtungsapparatur und reichert somit eine Feuchtigkeit an. Der Auslass der Befeuchtungsapparatur wird über einen Schlauch mit einem Feuchtesensor verbunden, der die aktuelle Luftfeuchte des Luftstroms analysiert. Der Feuchtesensor wird über einen Schlauch mit dem Adapter für den Respimat® Inhalator verbunden. Dieser wird an den modifizierten Sample Induction Port angeschlossen. Der modifizierte Sample Induction Port wird in die Messzone des Partikelgrößenmessgerät eingebaut und stellt die eigentliche Messkammer dar. Zur Erzeugung des Luftstroms wird der Auslass des modifizierten Sample Induction Ports über einen geeigneten Adapter an eine Absaugapparatur angeschlossen, wobei eine Auffangapparatur dazwischen geschaltet wird, in der die bei der Messung abgegebene Dosis aufgefangen wird. Die Absaugapparatur wird an eine Vakuumquelle angeschlossen.

Zur Bestimmung der Teilchengrößenverteilung bzw. des mittleren Volumendurchmessers wird das Partikelgrößenmessgerät mit einer R3 - Linse, Brennweite 100 mm, betrieben. Als Triggerbedingung zur Messung wird eine optische Konzentration von > 0,1% auf Kanal 30 eingestellt. Zur Auswertung wird die MIE - Theorie mit den dazugehörenden Stoffparametern verwendet.

### Durchführung der Messung:

Der Respimat® Inhalator wird gespannt, Mundstück, Überwurfmutter und Düse trocken gewischt und anschließend in den Adapter eingesetzt. Die Feuchtezuführung wird angeschlossen, und bei einer konstanten Luftfeuchtigkeit von ca. 100 % r.F. eine Referenzmessung durchgeführt. Durch Betätigen der Auslösetaste am Respimat® Inhalator wird des Aerosol generiert. Das abgegebene Aerosol wird vom Partikelgrößenmessgerät detektiert und dessen Teilchengrößenverteilung bzw. der mittlere Volumendurchmesser berechnet.

Die nachstehende Tabelle fasst die für die vorstehend genannten Formulierungsbeispiele bestimmten Tröpfchengrößen zusammen.

| **Beispiel** | **D(v;50)*** |
|---|---|
| A.1 | 2,0 ± 0,2 |
| A.2 | 2,3 ± 0,4 |
| A.3 | 1,6 ± 0,2 |
| A.4 | 2,0 ± 0,4 |
| A.5 | 2,0 ± 0,4 |
| A.6 | 2,0 ± 0,4 |
| A.7 | 2,0 ± 0,4 |
| A.8 | 1,6 ± 0,2 |
| B.1 | 2,1 ± 0,4 |
| A.9 | 2,0 ± 0,3 |
| A.10 | 2,3 ± 0,2 |
| B.2 | 1,7 ± 0,2 |
| B.3 | 2,1 ± 0,2 |
| B.4 | 1,6 ± 0,2 |
| B.5 | 2,1 ± 0,2 |
| B.6 | 2,3 ± 0,2 |
| B.7 | 2,1 ± 0,1 |
| C.1 | 2,3 ± 0,3 |
| C.2 | 2,1 ± 0,4 |
| C.3 | 2,1 ± 0,4 |

| | |
|---|---|
| *D(v;50) bedeutet: mittlerer Volumendurchmesser; | |

Demgegenüber wurden für Formulierungen, ohne zusätzlichen inerten Hilfsstoff nachstehende Werte bestimmt:

| Beispiel | Ethanol (%) | BAC (mg) | EDTA (mg) | Wirkstoff (%) | D(v;50) |
|---|---|---|---|---|---|
| Rev.1 | 0 | 10 | 50 | Tiotropium (0,1) | 0,5 ± 0,1 |
| Rev.2 | 0 | 10 | 10 | Tiotropium (0,045) | 0,4 ± 0,2 |
| Rev.3 | 95 | 10 | 10 | Fenoterol (1,0) | 0,9 ± 0,4 |

## Patentansprüche

1. Verwendung einer Lösungsformulierung zur Herstellung eines Arzneimittels zur Behandlung von Atemwegserkrankungen,
wobei die Lösungsformulierungen neben einem oder mehreren Wirkstoffen aus der Gruppe bestehend aus Anticholinergika, Betamimetika, Corticosteroide, PDE4-Inhibitoren, LTD4-Antagonisten, Dopamin-Agonisten und H1-Antihistaminika in einem Lösungsmittel ausgewählt aus der Gruppe Ethanol oder Mischungen aus Ethanol und Wasser, wenigstens einem inerten, nichtflüchtigen Hilfsstoff, ausgewählt aus der Gruppe bestehend aus Natriumchlorid, Magnesiumsulfat, Natriumhydrogenkarbonat, Kaliumhydrogenkarbonat und Magnesiumchlorid in einer solchen Menge enthalten, dass die Gesamtkonzentration an nichtflüchtigen Bestandteilen in der Formulierung ≥ 3 Gew.-% beträgt.

2. Verwendung von Lösungsformulierungen nach Anspruch 1, **dadurch gekennzeichnet, dass** die Gesamtkonzentration an nichtflüchtigen Bestandteilen in der Formulierung ≥ 5 Gew-%, bevorzugt ≥ 10 Gew-% beträgt.

3. Verwendung von Lösungsformulierungen nach Anspruch 1 oder 2, **dadurch gekennzeichnet, dass** sie als Lösungsmittel Mischungen aus Ethanol und Wasser enthalten, bei denen der prozentuale Massenanteil von Ethanol im Bereich zwischen 5 und 99 %, bevorzugt im Bereich von 10 bis 96 % Ethanol liegt.

4. Verwendung von Lösungsformulierungen nach einem der Ansprüche 1 bis 3, **dadurch gekennzeichnet, dass** der pH-Wert der erfindungsgemäßen Formulierung in einem Bereich von 2,0 und 6,5 liegt.

## Claims

1. Use of a solution formulation for preparing a pharmaceutical composition for the treatment of respiratory diseases, wherein the solution formulations contain, in addition to one or more active substances selected from the group consisting of anticholinergics, betamimetics, corticosteroids, PDE4-inhibitors, LTD4-antagonists, dopamine agonists and H1-antihistamines in a solvent selected from the group consisting of ethanol or mixtures of ethanol and water, at least one inert, non-volatile additive selected from the group consisting of sodium chloride, magnesium sulfate, sodium hydrogen carbonate, potassium hydrogen carbonate and magnesium chloride in such an amount that the total concentration of non-volatile constituents in the formulation is ≥ 3 wt.%.

2. Use of solution formulations according to claim 1, **characterised in that** the total concentration of non-volatile constituents in the formulation is ≥ 5 wt.%, preferably ≥ 10 wt.%.

3. Use of solution formulations according to claim 1 or claim 2, **characterised in that** said formulations contain, as solvent, mixtures of ethanol and water, wherein the percentage by weight of ethanol is in the range of between 5 and 99%, preferably in the range of 10 to 96% ethanol.

4. Use of solution formulations according to any one of claims 1 to 3, **characterised in that** the pH of the formulation according to the invention is in the range from 2.0 to 6.5.

## Revendications

1. Utilisation d'une formulation de solution pour fabriquer un médicament pour son utilisation dans le traitement de maladies respiratoires,
dans laquelle les formulations de solution contiennent outre un ou plusieurs principes actifs issus du groupe constitué des anticholinergiques, des bêta-mimétiques, des corticostéroïdes, des inhibiteurs de PDE4, des antagonistes de LTD4, des agonistes de la dopamine et des antihistaminiques H1 dans un solvant choisi parmi le groupe de l'éthanol ou des mélanges composés d'éthanol et d'eau, au moins un adjuvant inerte non volatile, choisi parmi le groupe constitué de chlorure de sodium, de sulfate de magnésium, d'hydrogénocarbonate de sodium, d'hydrogénocarbonate de potassium et de chlorure de magnésium, en une quantité telle que la concentration totale de constituants non volatiles dans la formulation est ≥ 3 % en poids.

2. Utilisation de formulations de solution selon la revendication 1, **caractérisée en ce que** la concentration totale de constituants non volatiles dans la formulation est ≥ 5 % en poids, de préférence ≥ 10 % en poids.

3. Utilisation de formulations de solution selon la revendication 1 ou 2, **caractérisée en ce qu'**elles contiennent en tant que solvants des mélanges composés d'éthanol et d'eau, dans lesquels la proportion en poids exprimée en pourcentages d'éthanol se situe dans la plage entre 5 et 99 %, de préférence dans la plage de 10 à 96 % d'éthanol.

4. Utilisation de formulations de solution selon l'une quelconque des revendications 1 à 3, **caractérisée en ce que** le pH de la formulation selon l'invention se situe dans une plage de 2,0 à 6,5.
